# EUROPÄISCHE PATENTANMELDUNG

(11) **EP 3 964 049 A1**
(43) Veröffentlichungstag der Anmeldung: **09.03.2022**
(21) Anmeldenummer: 20194195.2
(22) Anmeldetag: 02.09.2020
(51) Int. Cl.: A01G 9/02, A01H 4/00, A01G 9/029, A01G 31/06

(54) **LAGERBEHÄLTNIS, WACHSTUMS- UND/ODER VERMEHRUNGSSTATION, KULTIVIERUNGSSYSTEM UND VERFAHREN ZUR KULTIVIERUNG VON ENTWICKLUNGSGUT**

(71) Anmelder: Capsero AG, 4142 Münchenstein (CH)
(72) Erfinder: RUTZ, Benjamin, 4410 Liestal (CH); DÜRR, Günter, 4411 Seltisberg (CH); CIRILLO, Fabio, 4466 Ormalingen (CH)
(74) Vertreter: Specht, Peter

(57) **Zusammenfassung**

Ein Lagerbehältnis (1) umfassend einen Innenraum (3) und ein darin angeordnetes Substrat (4), welches ortsfest an der Wandung des Lagerbehältnisses angeordnet ist, wobei das Lagerbehältnis (1) zudem ein im oder am Substrat (4) angeordnetes biologisches lebendes Entwicklungsgut (6) in Form von Pflanzen, Pilzen und/oder Algen aufweist, wobei das Lagerbehältnis (1) als eine zumindest flüssigkeitsdicht- oder feststoffdicht-abgeschlossene Kapsel zur Gewährleistung einer aseptischen Atmosphäre, insbesondere eine Aseptik nach ISO 11135, ISO 11137, ISO 17665-1, ISO 13408-1, EU GMP Annex 1 oder US cGMP mit einem Sterilisationsprozessziel eines SAL (sterility assurance level) von mindestens 10⁻⁵, ausgebildet ist, sowie eine Wachstums- und/oder Vermehrungsstation, ein Kultivierungssystem und ein Verfahren zur Kultivierung von Entwicklungsgut.

## Beschreibung

Die vorliegende Erfindung betrifft ein Lagerbehältnis, eine Wachstums- und/oder Vermehrungsstation, ein Kultivierungssystem und ein Verfahren zur Kultivierung von Entwicklungsgut

In der Schrift JP 4'979'976 B2 wird ein automatisierter, aseptischer Kultivierungsraum beschrieben. Diese Technologie hat den Nachteil, dass aufgrund der technischen Installationen der Raum nicht maximal für Bepflanzungen genutzt werden kann und das Pflanzgut nicht aseptisch eingebracht werden kann. Zudem sind kommerziell verpackte inkrustierte Samen erhältlich, welche jedoch ebenfalls nicht in einer aseptischen Atmosphäre und in einer definierten Position der Verpackung angeordnet sind.

Weiterhin sind aus dem Stand der Technik JP 4'979'976 B2 Gewächshäuser bekannt, in welchen eine mittels Ernterobotern eine Ernte unter aseptischen Bedingungen durchgeführt wird. Weiter sind aus der JP 4'979'976 B2 begehbare Behältnisse zur Zucht von Pflanzen oder ähnlichen Gütern bekannt. Diese weisen allerdings keinerlei Vorkehrungen für eine aseptischen Prozessierung nach der Ernte auf.

Ausgehend vom vorliegenden Stand der Technik ist es die Aufgabe der vorliegenden Erfindung Entwicklungsgut möglichst so zur Kultivierung zu geben, dass das Entwicklungsgut während der Einbringung, Entwicklung, Ernte und Prozessierung nicht verunreinigt wird. Weiter soll das Entwicklungsgut so bereitgestellt werden, dass diese geschützt transportierbar ist, welche den Transportzeitraum bereits zur Kultivierung nutzt, welche gut lagerbar ist und modular im Rahmen einer Farmhaltung erweiterbar ist. Insbesondere entsteht diese Aufgabe den Anforderungen der regulierten Industrien, wie Pharmazie, Kosmetik und Nahrungsergänzungsmitteln.

Die vorliegende Erfindung löst die Aufgabe durch ein Lagerbehältnis mit den Merkmalen des Anspruchs 1, sowie durch eine Wachstums- und/oder Vermehrungsstation mit den Merkmalen des Anspruchs 9, sowie einer Maintenance-Einheit des Anspruchs 13, sowie ein Verfahren des Anspruchs 14.

Ein erfindungsgemäßes Lagerbehältnis umfasst einen Innenraum und ein darin angeordnetes Substrat. Das Substrat bildet die Oberfläche, welches ein Wachstum oder eine Vermehrung von biologischen lebenden Entwicklungsgut erlaubt. Insbesondere kann das Substrat eine Verwurzelung, also eine Durchdringung oder ein Anhaften von Wurzeln, erlauben. Poröse Materialien, wie z.B. poröses Gestein oder ein Schwamm-Material, oder gelartige Materialien, wie z.B. Hydrogele oder Gele aus biologischen Materialien, sind dafür besonders gut geeignet. Besonders bevorzugt weist das Entwicklungsmaterial eine hohe Wasseraufnahmekapazität auf. Anstelle von Wasser können selbstverständlich auch andere flüssige Medien, z.B. eine Nährstofflösung aufgenommen werden. Nährstoffe können z.B. in flüssiger aber auch in fester Form, z.B. als Salz, im Substrat enthalten sein.

Das Substrat kann sich vorzugsweise über die gesamte Breite des Innenraumes erstrecken und ist erfindungsgemäß ortsfest an der Wandung des Lagerbehältnisses angeordnet, z.B. durch Kraftschluss oder z.B. durch Auflage des Substrats auf einer Netzlage, die senkrecht zur Längsachse des Lagerbehälters angeordnet ist. Alternativ oder Zusätzlich kann das Substrat auch der Gestalt ausgebildet sein, dass ohne eine Auflagestruktur das Substrat ortsfest durch Adhäsion an der Wandung des Lagerbehältnisses und durch Kohäsion im Substrat selber besteht. Ebenfalls bevorzugt kann das Substrat als ein saugfähiges Material ausgebildet sein mit einer Wasseraufnahmekapazität im trockenen Zustand von zumindest 50 g Wasser/cm³.

Das Lagerbehältnis weist erfindungsgemäß zudem ein im oder am Substrat angeordnetes biologisches lebendes Entwicklungsgut auf. Das Entwicklungsgut kann vorzugsweise eine Pflanze, z.B. in Form eines Samens, eines Keimlings oder eines klonierten Setzlings, sein. Es können aber auch Pilze, Pilzsporen, Algen, insbesondere Einzeller oder Mehrzeller, oder andere biologische Organismen als Entwicklungsgut eingesetzt werden. Weitere Organismen, welche unter den Begriff "Entwicklungsgut" fallen, werden in der nachfolgenden Beschreibung genannt.

Insbesondere bei Pflanzen würde der Fachmann für ein Lagerbehältnis eine offene Vorrichtung, z.B. einen Blumentopf oder dergleichen vorsehen, da die Pflanze bei längerer Aufbewahrung schimmeln kann, eine Wasserzufuhr nicht möglich ist und auch ansonsten keine hinreichende Nährstoffversorgung gewährleistet ist.

Auch andere biologische Organismen (z.B. Bakterien oder Wasserlebewesen) können im Lagerbehältnis im Rahmen der vorliegenden Erfindung aseptisch gehalten werden. Das Lagerbehältnis kann z.B. mit einer sterilen Nährlösung unter sterilen bzw. aseptischen Bedingungen befüllt und das Entwicklungsgut unter aseptischen Bedingungen eingebracht werden und die Kapsel sodann hermetisch und aseptisch verschlossen. Die Nährlösung kann hierbei geeignete Nährstoffe um das Entwicklungsgut unter Ausschluss von äußeren Fremdeinflüssen am Leben zu erhalten, enthalten. Derartige Fremdeinflüsse sind z.B. Überbelichtung und/oder unerwünschte Fremdgase. Eine gasdicht-abgeschlossene Kapsel oder Kapsel mit einem gaspermeablen, insbesondere gasselektiven, Wandsegment unter Ausschluss von Fremdgasen, Flüssigkeiten und/oder Feststoffen, wie Keimen, verhindert dies. Entsprechend kann auch eine transparente Kapsel mit einer Beschichtung, z.B. einer UV-inhibierenden Schicht, ausgestattet sein, um eine Überbelichtung zu verhindern.

Das Lagerbehältnis ist allerdings entgegen dieser Überlegung erfindungsgemäß als eine abgeschlossene Kapsel ausgebildet, wobei das Lagerbehältnis vorzugsweise ein rohrförmiges Mittelsegment aufweist, welches die Längsachse definiert. Die Kapsel soll nur den kurzen Zeitraum zwischen der Bereitstellung der Pflanze, z.B. einem Klonierungsprozess, und dem Einsetzen der Pflanze in eine aseptische Atmosphäre eines Containers überbrücken. Der Aufenthalt des Entwicklungsgutes im Lagerbehältnis im geschlossenen Zustand ist daher vorzugsweise weniger als 14 Tage, insbesondere weniger als 5 Tage. Sofern sich das Lagerbehältnis in der aseptischen Atmosphäre befindet, kann dieses geöffnet werden, wodurch eine Wasserzufuhr und ein Luftaustausch möglich wird und ein Schimmeln verhindert wird.

Das Lagerbehältnis gewährleistet somit eine aseptische Atmosphäre, bevorzugterweise eine Aseptik nach ISO 11135, ISO 11137, ISO 17665-1, ISO 13408-1, EU GMP Annex 1 oder US cGMP mit einem Sterilisationsprozessziel eines SAL (sterility assurance level) von mindestens 10⁻⁵. Die vorgenannten Richtlinien beziehen sich auf die jeweilige Fassung zum Prioritätszeitpunkt der vorliegenden Anmeldung. Besonders bevorzugt besteht das Lagerbehältnis aus einem formstabilen Material, welches in eine Öffnung eines Lagergestells einsetzbar ist.

Vorteilhafte Ausgestaltungen der Erfindung sind Gegenstand der Unteransprüche.

Das Lagerbehältnis kann zumindest ein Endsegment, vorzugsweise zwei Endsegmente und ein rohrförmiges Mittelsegment aufweisen, wobei das Substrat und das Entwicklungsgut, insbesondere die Pflanze, im Mittelsegment angeordnet ist und wobei zumindest eines der Endsegmente gegenüber dem Mittelsegment eine geringere Wandstärke aufweist oder ein gegenüber dem Mittelsegment ein thermisch- und/oder chemisch-angreifbareres formstabiles Wandungsmaterial aufweist. Formstabil bedeutet im Zusammenhang der vorliegenden Erfindung, dass das Wandmaterial, anders als bei einer Folie, nicht faltbar ist, sondern einen Behälter, also einen Formkörper ausbildet. Eine Verformbarkeit der Wandung, z.B. bei Kunststoffkapsel, ist allerdings im Rahmen der vorliegenden Erfindung möglich.

Um zumindest einen Gasaustausch im geschlossenen Zustand des Lagerbehältnisses zu gewährleisten kann zumindest eines der Endsegmente aus einem gaspermeablen, insbesondere gasselektiv-durchlässigen Material gebildet sein. Dies kann u.a. durch die Polarität des Materials und durch die Gaspermeabilitätskonstante des Materials bestimmt werden.

Eine Wandung des Lagerbehältnisses, insbesondere des Mittelsegments, kann zumindest bereichsweise oder vollständig aus einem transparenten Material bestehen, so dass eine visuelle Kontrolle über den Zustand des Entwicklungsgutes im Lagerbehältnis möglich ist.

Für eine definierte Position des Lagerbehältnisses in einer Öffnung eines Lagergestells kann das Mittelsegment zumindest einen umfangsverteilten Begrenzungsanschlag oder mehrere umfangsverteile Begrenzungsanschläge als Axialanschlag oder eine konische Form für den gleichen Zweck aufweisen.

Das Lagerbehältnis kann zudem eine allumfassende sterilisierbare Oberfläche aufweisen, die durch geeignete Sterilisationsmethoden, wie z.B. UVC, Ethanol, Ozon, Perchloressigsäure, Wasserstoffperoxid, usw. sterilisierbar ist. Die Sterilisation kann sowohl als CIP als auch als WIP, Wischsterilisation, Tauchsterilisation, Belichtung oder Begasung durchgeführt werden. Insbesondere eignen sich hierzu Materialien des Lagerbehältnis welche auf Basis eines Metalles, wie Aluminium, Eisen, von Glas, eines Thermoplastes, wie z.B. Polyamid, Polycarbonat, Polyolefin, Polyacryl, Polymethacryl, halogenierte Ethylene, usw. und/oder eines Elastomeren, wie z.B. Kautschuk, halogenierte Elastomere, Silikone, usw. gebildet ist oder zu mindestens 50% aus diesen bestehen.

Weiterhin erfindungsgemäß ist eine Wachstums- und/oder Vermehrungsstation zur Kultivierung von Entwicklungsgut, wobei die Wachstums- und/oder Vermehrungsstation als ein Großraumbehälter, insbesondere als ein ISO-Container, ausgebildet ist und wobei der Großraumbehälter eine Vorrichtung zur Erzeugung und/oder Überwachung einer aseptischen Innenatmosphäre aufweist.

Die Innenatmosphäre kann sich lediglich in einem Teilbereich des Großraumcontainers, insbesondere einem Wachsraum in welchem das Entwicklungsgut gelagert ist, beschränken. Ein zum Wachsraum benachbarter Technikraum hingegen kann auch nicht unter dieser Innenatmosphäre stehen und somit unter Aufrechterhaltung der aseptischen Bedingungen von außen zugänglich sein.

Vorteilhaft weist die Wachstums- und/oder Vermehrungsstation einen Wachsraum auf mit einer Zu- und/oder Ableitung eines CIP-Mediums, insbesondere von UV-C Strahlung, Gasen, wie Ozon, Ethylenoxid, Propylenoxid, oder eines vernebelten flüssigen Mediums, wie Wasserstoffperoxid, anorganische Säuren oder anorganische Basen. Bei Gasen und Nebel ist es zudem bevorzugt, dass ein Verteilsystem vorhanden ist um den gesamten Innenraum zu dekontaminieren. Dies kann insbesondere mit Ventilation oder druck-geförderten vernebelten Medien geschehen. Insbesondere ist für Gase eine Ventilationsverteilung und bei Fluiden Sprühdüsen vorteilhaft.

Die Überwachung der erfolgreichen Dekontamination wird durch geeignete Sensorik gemessen, wie z.B. Gassensoren zur Ermittlung der Gaskonzentration, Strahlungssensoren zur Ermittlung des Photonenflusses, pH-Sensoren zur Ermittlung der Säure- oder Basenstärke, und/oder ionenselektive Elektronen zur Ermittlung der lonenstärke. Im Zusammenhang mit der Konzentration muss die Einwirkzeit gemessen werden um eine erfolgreiche Dekontamination zu bestätigen.

Bei der Dekontamination wird vorteilhaft Ozon verwendet, dass durch Lichtbogen-Entladung oder durch UV-C an Luftsauerstoff gebildet wird und solange in den Wachsraum eingeleitet wird, bis die Konzentration und/oder die Einwirkzeit erreicht wurde.

Nach erfolgreicher Dekontamination wird das Überschüssige CIP-Medium aus dem Wachsraum abgeführt um das Entwicklungsgut nicht zu schädigen. Hierfür können geeignete Massnahmen ein Belüften mit Frischluft oder Prozessgas, wie Stickstoff, Kohlendioxid, Argon und/oder Spuren davon in Luft, Niederwaschen oder eine Titration sein. Vorteilhaft ist ein Niederwaschen mit Wasser, mit anschließender Neutralisierung.

Weiterhin kann der Großraumbehälter zumindest ein Lagergestell zur Anordnung eines oder vorzugsweise einer Mehrzahl von erfindungsgemäßen Lagerbehältern aufweisen. Bevorzugt ist das oder sind die Lagergestelle bewegbar, insbesondere verfahrbar, gegenüber der Containerwand angeordnet. Hierfür können die Lagergestelle rollengelagert, pressluftgelagert und/oder gleitlagergelagert sein.

Weiterhin bevorzugt kann der erfindungsgemäße Großraumbehälter zumindest eines, mehrere oder vorzugsweise sämtliche der folgenden Vorrichtungen aufweist:
- eine Lichtquelle zur Bestrahlung des Entwicklungsgutes
- eine Pumpe zum Medientransport, einschließlich einem nährstoffhaltigen Medium und/oder einem Sterilisationsmedium;
- einen Sensor zur Überwachung des Gesundheitszustands des Entwicklungsgutes;
- eine Steuer- und/oder Auswerteeinheit zur Steuerung eines Regelkreises zur Anpassung der Innenatmosphäre an die vom Sensor erfassten Änderungen;
- eine Wasserversorgungs- und/oder Aufbereitungseinheit;
- ein Klimagerät;
- ein Nährstoffbehälter;
- eine Energieversorgungs- und/oder -verwaltungseinheit;
- eine Mischkammer und/oder
- ein Kommunikationsmodul zum Datenaustausch mit einem externen Gerät.

Einzelne Vorrichtungen, insbesondere Elektronikvorrichtungen, können in einem vom Wachsraum räumlich-getrennten Servicebereich angeordnet sein, welcher von außen zugänglich ist und nicht der aseptischen Atmosphäre unterliegt.

Medien- und/oder Signal- oder Stromleitungen von diesem Servicebereich in den Wachsraum sind vorzugsweise mediendicht in einer Trennwand angeordnet, insbesondere vergossen, so dass die Innenatmosphäre nicht in den Serviceraum eindringen kann und umgekehrt die Aseptik im Wachsraum auch bei Zugang des Servicebereichs erhalten bleibt. Weitere vorteilhafte Ausgestaltungen der Wachstums- und/oder Vermehrungsstation ergeben sich aus der nachfolgenden Beschreibung und den Figuren.

Weiterhin erfindungsgemäß ist ein Kultivierungssystem umfassend eine Wachstums- und/oder Vermehrungsstation sowie eine Maintenance-Einheit, insbesondere eine Ernteeinheit, wobei die Maintenance-Einheit mit der Wachstums- und/oder Vermehrungseinheit zur Überführung von Entwicklungsgut oder Maschinen zwischen den beiden Einheiten verbunden ist und wobei die Maintenance-Einheit die aseptische Innenatmosphäre aufweist.

Die Maintenance-Einheit kann Teil des Containers sein, welcher auch den Wachsraum aufweist oder, besonders bevorzugt für eine größere modulare Anordnung, kann die Maintenance Einheit in einem gesonderten Container angeordnet sein. Eine Schleuse kann zwischen zwei Containern angeordnet und mit geeigneten Maßnahmen, wie z.B. Unterdruck, insbesondere Vakuum, oder wie oben genannt eine oder mehrere CIP-Medien, dekontaminiert werden. Durch Öffnen beider Container kann die Schleuse mit der aseptischen Innenatmosphäre geflutet werden. Dies stellt allerdings nur eine Variante der Kopplung der beiden Container dar. Eine Doppeltür an einem oder beider der beiden Container kann ebenfalls eine Sicherheitsvorrichtung zur Kopplung der Container darstellen. Die Container weisen dabei geeignete Kopplungsvorrichtungen auf, um die beiden Container in benachbarter Position fest und, vorzugsweise mediumsdicht, miteinander zu verbinden.

Weiterhin erfindungsgemäß ist ein Verfahren zur Kultivierung von Entwicklungsgut, welches zumindest die folgenden Schritte aufweist:
a) Bereitstellen eines erfindungsgemäßen Lagerbehälters,
b) Einbringung eines Entwicklungsgutes in den Lagerbehälter unter aseptischen Bedingungen.
c) Einsetzen des Lagerbehälters in ein Lagergestell einer erfindungsgemäßen Wachstums- und/oder Vermehrungsstation und zumindest einseitig-endständiges Öffnen des Lagerbehälters. Bevorzugt kann der Behälter allerdings auch beidseitig endständig geöffnet werden.
   Die Endsegmente können mit dem Mittelsegment über Verbindungsmittel und/oder Abdichtmittel verbunden sein. Derartige Verbindungsmittel können rein mechanische Mittel wie Rast- oder Klemmmittel und Dichtringe oder z.B. auch mit den Mittel- und Endsegmenten stoffschlüssig verbundene Kunststoffbereiche oder folienartige Kunststoffumwickelungen an den Schnittstellen der Mittel- und Endsegmente sein, welche sich unter bestimmten Bedingungen auflösen.
   Die Verbindungs- und/oder Abdichtmittel können somit auf mechanischem und/oder besonders bevorzugt auf chemischem Wege lösbar sein. Das Verbindungsmittel ist dichtend und umfänglich jeweils an einer Schnittstelle bzw. Übergang zwischen Mittel- und Endsegment um das Lagerbehältnis angeordnet.
   Vorteilhaft kann die Schnittstelle oder der Übergang zudem derart ausgebildet sein, dass sich beim Lösen des jeweiligen Verbindungs- und/oder Abdichtmittels eine selbständige Trennung von Mittel- und Endsegment ergibt. Dies kann z.B. durch eine schiefe Ebene an der Schnittstelle beider Segmente ergeben, welche ein Abrutschen des Endsegments nach dem Lösen des Verbindungs- und/oder Abdichtmittels ermöglicht. Es können auch andere geometrische Ausbildungen vorgesehen sein, welche ein selbständiges insbesondere schwerkraftbedingtes Trennen der beiden Segmente ermöglichen, so dass durch ein Lösen des Verbindungs- und/oder Abdichtmittels ein gleichzeitiges Lösen der Endsegmente gegenüber dem Mittelsegment erfolgt.
d) Überwachen des Wachstums und/oder der Vermehrung des Entwicklungsgutes in der aseptischen Innenatmosphäre, bis zur Detektion eines Wachstums- und/oder Vermehrungsstadiums für die Ernte;
   Das Wachstums- oder Vermehrungsstadium kann durch optische Analyse, z.B. der Größe der Pflanze, der Ausbreitung eines Bakterienstamms oder das Ausgasen von organischen Stoffen, z.B. anhand der Konzentration an VOC's, wie Terpenen, die nur oder vemehrt bei der Erntezeit auftreten, ermittelt werden und mit einem Sollwert abgeglichen werden. Allerdings sind auch andere Messverfahren im Rahmen der vorliegenden Erfindung möglich.
e) Einsatz einer Maintenance-Einheit unter Aufrechterhaltung der aseptischen Atmosphäre unter Gewinnung eines Wertstoffs, insbesondere eines wirkstoffhaltigen biologischen Materials.

Sodann wird eine Maintenance-Einheit, vorzugsweise eine erfindungsgemäße Maintenance-Einheit, zur Gewinnung des Wertstoffs eingesetzt.

Bevorzugt erfolgt in Schritt c), vorzugsweise nach dem Einsetzen des Lagerbehälters, eine Sterilisation, vorzugsweise im Rahmen eines CIP-Verfahrens. Dies impliziert, dass das Lagerbehältnis zumindest während der Sterilisation den Sterilisationsbedingungen standhält und somit das Entwicklungsgut schützt.

Weitere vorteilhafte Ausgestaltungsvarianten der Erfindung, sowohl des Verfahrens als auch der Vorrichtungen werden nachfolgend beschrieben.
Ein erster Prozessschritt des Verfahrens kann u.a. das Einbringer von Entwicklungsgut in das Lagerbehältnis zusammen mit Substanzen sein, die das Wachstum beeinflussen können.

Das Entwicklungsgut können einzelne Setzlinge oder irgendeine Form entwicklungsfähiger, insbesondere lebender, Biomasse sein.
Das Lagerbehältnis kann dem Entwicklungsgut sowohl als Lager-, Manipulations- oder Transportschutz dienen, insbesondere bei Boden-, Wasser- oder Lufttransport.

Das Lagerbehältnis ist im Prozess sterilisierbar und kann, vorzugsweise nach erfolgter Einsetzung in Tablare des Lagergestells, und nach Sterilisierung der Innenatmosphäre automatisch geöffnet werden.

Weiterhin kann das Lagerbehältnis stabil in die Tablare verankert werden und durch eine auf dem Lagerbehältnis angeordnete Markierung eindeutig identifizierbar werden.

Weiterhin kann eine einzelne Wachstums- und/oder Vermehrungsstation oder mehrere solcher Stationen, die zu einer sogenannten Farm zusammengefasst sind, einen Servicebereich, nachfolgend auch Prozessraum genannt, aufweisen und eine Maintenance-Einheit.

Die Wachstums- und/oder Vermehrungsstation oder die Farm kann eine durchgehend aseptische Kultivierung von zu kultivierender Biomasse, Entwicklungsgut genannt, zulassen, ohne dass ein Mensch in das System eintreten muss oder darf.

Die Wachstums- und/oder Vermehrungsstationen sind stapelbar und weisen typisch für ISO-Container, insbesondere in den Eckbereichen, Anschläge zur Stapelung auf.

Die Wachstums- und/oder Vermehrungsstation oder die Farm kann Innenatmosphärebedingungen in der Einheit (Innenatmosphäre genannt) aufweisen, die entsprechend den Bedürfnissen des Entwicklungsgutes vorzugsweise wie folgt zur Verfügung gestellt werden können, indem
i) die Innenatmosphärebedingungen in der Einheit als closed-loop aufgesetzt sind, und dass
ii) die atmosphärischen Bedingungen sich kontrollieren lassen und die aseptischen Kriterien eines Reinraumes nach ISO und/oder GMP erfüllen,
iii) dem Entwicklungsgut die Temperatur, die Feuchte, die Nährstoffzusammensetzung, der Druck, das elektromagnetische Feld, der Luftstrom, die Atmosphärenzusammensetzung, der pH-Wert, die Lichtstärke, das Lichtspektrum, und/oder der Tag/Nacht-Zyklus bedarfsentsprechend zur Verfügung gestellt werden können,
iv) die Bedingungen in der Innenatmosphäre just in time gemessen und aufgezeichnet werden,
v) die aufgezeichneten Daten lokal und/oder zentral zur Analyse und Interpretation herangezogen, aufbereitet und zur Steuerung der weiteren Prozesse verwendet werden.

Die Wachstums- und/oder Vermehrungsstation oder die Farm kann die Masse und transporttechnischen Eigenschaften von mit Lastwagen oder Schiffen transportierbaren Normcontainern (ISO-Containern) aufweisen.

Die Wachstums- und/oder Vermehrungsstation oder die Farm kann zum Betrieb mindestens eine Stromversorgung und ein Kommunikationsmodul, insbesondere ein Funkmodul, aufweisen.

Die Einheit mit einem anbauflächenoptimierten Lagergestellen bzw. Regalsystem kann aus zumindest zwei parallel zueinander verschiebbaren Regalen mit je in den Regalen integriertem Versorgungssystem, gebildet sein.

Alle flüssigen Medien, teilweise auch die gasförmigen Medien und die Energie, die Sensoren und ihre Anbindung können in den Hohlräumen der Regalsystemen geführt werden.

Das Regalsystem lässt zur Kultivierung des Kultivierungsgutes die Förderung von Nährstoffen zu. Insbesondere können die Nährstoffe sowohl aeroponische als auch hydroponische, oder eine Mischung dieser, an das Kultivierungsgut gefördert werden.

Eine aeroponische Kultivierung kann vorzugsweise von einem Niedrigdrucksystem durch Vernebelung mittels Piezoelementen bewerkstelligt werden.

Eine hydroponische Kultivierung kann mittels Niedrigdrucksystem, insbesondere durch Nährstoff-Film-Technologie bewerkstelligt werden.

Die einzelnen Regale können eines oder mehrere Tablare für die Aufnahme von Lagerbehältnissen enthalten, die den Lagerbehälter oder eine Mehrzahl von Lagerbehältern aufnehmen können. Die Tablare sind modular, wiederverwendbar, reinigbar und sterilisierbar, die mit der Möglichkeit einer festen Verankerung des Lagerbehältnis versehen sind.

Die Maintenance-Einheit erlaubt den aseptischen Transfer von einem Entwicklungsgut aus dem Wachstums- und/oder Vermehrungsstation in die Maintenance-Einheit, insbesondere in Form einer Ernteeinheit.

Die Maintenance-Einheit ist vorzugsweise in horizontaler und vertikaler Richtung verfahrbar,
(a) für die Verfahrung in vertikaler Richtung kann eine Scheren-Hebung benutzt werden,
(b) für die Verfahrung in horizontaler Richtung kann ein Mecanum-Antrieb benutzt werden,

Einzelne Ausführungsvarianten der Erfindung werden nachfolgend anhand der beiliegenden Figuren näher beschrieben. Die Figuren zeigen lediglich bevorzugte Ausführungsvarianten und sind nicht beschränkend für den Gegenstand der vorliegenden Erfindung. Der Fachmann wird jedoch einzelne Elemente der jeweiligen Ausführungsvarianten auch auf weitere Ausführungsvarianten übertragen, so dass diese nicht nur im Kontext mit der konkreten Ausführungsvariante offenbart sind. Es zeigen:
- Fig. 1: geschnittene Seitenansicht durch ein Lagerbehältnis für lebendige, insbesondere wachstums- oder vermehrungsfähige, Biomasse;
- Fig. 2: geschnittene Ansicht in Längsrichtung der Wachstums- und/oder Vermehrungsstation;
- Fig. 3: Rückansicht der Wachstums- und/oder Vermehrungsstation;
- Fig. 4: geschnittene Seitenansicht durch eine Versorgungsstation zum Anschluss an die Wachstums- und/oder Vermehrungsstation;
- Fig. 5: geschnittene Seitenansicht durch eine erste Maintenance-Einheit zum Anschluss an die Wachstums- und/oder Vermehrungsstation; und
- Fig. 6: geschnittene Seitenansicht durch eine zweite Maintenance-Einheit zum Anschluss an die Wachstums- und/oder Vermehrungsstation.

Durch bekannte Klonierungs- oder anderweitige Reproduktions- oder Produktionsmethoden wird Biomasse hergestellt, die als Ausgangsprodukt geeignet ist, welches sich in einer anschließenden Wachstumsphase vermehrt und/oder entwickelt, z.B. wächst. Die Wachstumsphase wird im Folgenden auch Reifung genannt.

Die Biomasse kann sowohl der Gruppe der Prokarioten als auch der Gruppe der Eukarioten zugehören. Die Biomasse kann insbesondere mittels somatischer Embryogenese, zygotischer Embryogenese und/oder Apomixis und deren Untergruppen über eine Methode produziert werden, die sich dadurch auszeichnet, dass aseptische Bedingungen eingehalten werden können.

Als Startzellen im Falle von der Klonierung können Zellen aus dem Meristem, dem einfachen permanenten und/oder dem komplexen permanenten Gewebe stammen. Des Weiteren können Stammzellen, Sporen, Spermien, Eizellen und/oder Samen verwendet werden.

Weiter kann die Biomasse durch geeignete chemische, biologische und/oder physikalische Maßnahmen wie Hormone, Toxine, Enzyme und/oder durch Kühlung, Gefrierung oder Trocknung konserviert werden. Die Biomasse wird im Folgenden Entwicklungsgut genannt, weil die gewonnene Biomasse in den hier beschriebenen Prozessschritten I, II und III ihrer Entwicklung hin zur erntereifen Form zugeführt werden soll.

Das Entwicklungsgut wird in einem ersten Schritt unter aseptischen Bedingungen in eine Verpackung eingebracht, im Weiteren Lagerbehältnis genannt.

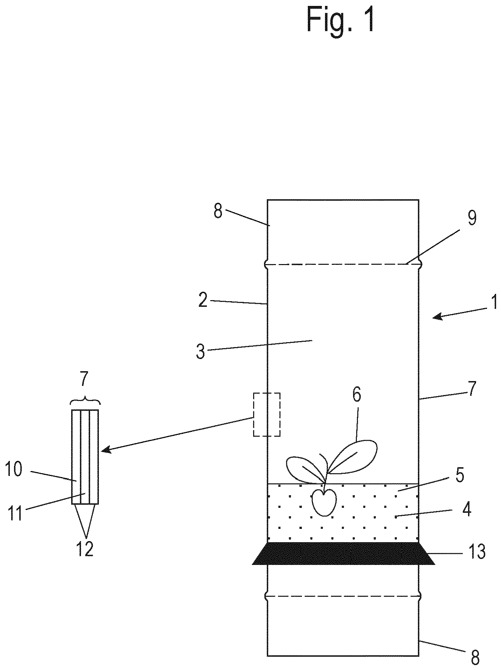

Fig. 1 zeigt ein Lagerbehältnis 1 für ein aseptisches Lagerbehältnis, welches hermetisch gegenüber der Umgebung abgeschlossen ist. Hierfür weist das Lagerbehältnis in der Ausführungsvariante im Bereich eines Mittelsegments 2 einen Innenraum 3 auf, in welchem ein Substrat 4 zur Bereitstellung und/oder Lagerung von Nährstoffen 5 für ein im Substrat 4 angeordnetes unter aseptischen Bedingungen gezüchtetes Entwicklungsgut 6, insbesondere einen Pflanzensamen, Pflanzensetzling oder ein Pflanzenkeim und/oder eine Pilzspore und/oder Alge oder dergleichen angeordnet ist. Ein solches Substrat kann u.a. Wasser oder hydrathaltiges Gel aufweisen, sowie verschiedene Salze, Hormone, Vitamine, Kohlenhydrate, Chelate und/oder Aminosäuren, welche das Pflanzenwachstum zusätzlich unterstützen.

Der Innenraum 3 wird durch eine Wandung 7 begrenzt, welche sich umfänglich in Form eines Rohres um das Substrat 4 erstreckt. Dabei weist das Mittelsegment 2 zu beiden Seiten ein Endsegment 8 auf, welches den Innenraum 2 endständig verschließt. Die Endsegmente 8 weisen am Übergangsbereich zum Mittelsegment 2 eine lösbare Verbindung, z.B. eine abgedichtete mechanische Schnittstelle und/oder eine Sollbruchstelle 9 auf.

Die mechanische Schnittstelle 9 kann als eine kraftschlüssige Verbindung des Endsegments mit dem Mittelsegment, z.B. mit einer umlaufenden Dichtung ausgebildet sein, wobei jeweils das End- und Mittelsegment über ein mechanischen Verbindungsmechanismus, z.B. einen Rastmechanismus, miteinander verbunden sind. Alternativ oder zusätzlich kann auch eine stoffschlüssige Verbindung vorgesehen sein, wie dies bei einer Sollbruchstelle der Fall ist, oder eine isolierende und/oder klebende Verbindung, wie dies bei z. B. einer Schmelzdichtung der Fall ist.

Je nach Variante kann die Schnittstelle zudem ein Filmscharnier aufweisen, welches nach Lösen des mechanischen Verbindungsmechanismus, das jeweilige Endsegment als deckelartige Ausgestaltung mit dem Mittelsegment 2 verbindet.

Alternativ oder zusätzlich kann das Lagerbehältnis 1 eine wasser- und/oder kontaminationsabweisende Schutzschicht 10 aufweisen, beispielsweise eine Wachsschicht. Die Beschichtung ermöglicht eine Oberflächensterilisierbarkeit im Rahmen eines CIP-Verfahrens oder eines beliebigen anderen Sterilisationsverfahrens und/oder hält die aseptische Barriere aufrecht

Hierfür weist die Wandung 7 des Lagerbehältnisses 1 eine Sperrschicht 11, vorzugsweise aus Glas, Polyolefin, Polyamid, halogenierten Polyvinylen, Therephthalaten und/oder EVOH auf, wobei die Diffusionssperrschicht vorzugsweise zumindest 2% der Wandstärke, vorzugsweise zwischen 10-100% der Wandstärke, einnimmt. Weiterhin kann die Diffusionssperrschicht zwischen zwei Stützschichten 12 angeordnet sein. Dabei kann es sich um einen beliebigen transparenten Stoff handeln, so dass der Zustand der Pflanzen innerhalb des Lagerbehältnisses visuell erkennbar ist.

Die Wandung 7 des Lagerbehältnisses 1 besitzt zusätzlich einen UV-Schutz in Form des Materials und/oder Materialzusätze, wie Polymeradditive der Reihe der Benzotriazole, Triazine, Acrylate, Phenone und/oder HALS und/oder als Sperrschicht 11 vorzugsweise aus UV-hemmenden Material, wie pigmentierte und/oder opaque Polymere,

Das Lagerbehältnis 1 weist eine bevorzugte Formstabilität, dass ein Transport des Lagerbehältnis und das darin befindliche Gut auf dem Boden-, Wasser- und Luftweg möglich ist. Insbesondere weist das Lagerbehältnis eine Formstabilität von einem Druckunterschied von mindestens 255 hPa auf, bevorzugterweise von 500 hPa.

Weiterhin weist das Lagerbehältnis 1 zumindest einen Begrenzungsanschlag 13 auf, welcher radial aus dem Mittelsegment 2 heraussteht und die Einstecktiefe des Lagerbehältnisses 1 in ein Lagergestell 14 in einer Wachstums- und/oder Vermehrungsstation 100 begrenzt.

Aufgrund der mechanischen Schnittstelle und/oder der Sollbruchstelle 9 ist das Lagerbehältnis 1 an einer definierten Position vorzugsweise automatisiert öffenbar. Somit kann die beidseitig-endständige Öffnung des Lagerbehältnisses 1 ohne menschliches Zutun in der aseptischen Atmosphäre einer nachfolgend beschriebenen und ebenfalls erfindungsgemäßen Wachstums- und/oder Vermehrungsstation 100 erfolgen.

Das Lagerbehältnis 1 ist insbesondere so geformt, dass es automatisiert am Ort der Reifung des Entwicklungsgutes 6 in die für die Reifung vorgesehene Öffnung des Lagergestells 14 einsetzbar ist. Die Öffnungen für die Reifung im Lagergestell 14 werden im Weiteren Setzplatz genannt. Die Phase zwischen Einbringung des Entwicklungsgutes 6 in das Lagerbehältnis 1 bis zur Einbringung des Lagerbehältnisses in den Setzplatz wird im Folgenden Prozessschritt II genannt.

Der Setzplatz ist ein Ort in einer Wachstums- und Vermehrungsstation 100, im Folgenden auch Wachsraum genannt, an dem das Lagerbehältnis 1 während der Reifung bis zur Ernte verbleibt und an dem das Entwicklungsgut 6 mittels des Lagerbehältnisses 1 stabil gehalten wird.

Das erfindungsgemäße Lagerbehältnis 1 ist erfindungsgemäß mit dem Entwicklungsgut 6 versehen, welches auf dem vorgenannten Substrat 4 platziert ist, welches ein Depot an Substanzen enthält, die das Wachstum bzw. die Vermehrung des Entwicklungsgutes 6 stoppen, hemmen, verzögern, verlangsamen, beschleunigen, fördern und/oder erlauben können.

Das Lagerbehältnis 1 ist so ausgelegt, dass es als eine sterile Barriere zwischen der Umgebung außerhalb der Wachstums- und Vermehrungsstation 100 und dem Entwicklungsgut 6 ausgebildet ist. Diese sterile Barriere bietet das Lagerbehältnis 1 sowohl während der Verpackung des Entwicklungsgutes 6, als auch während seiner Lagerung, seines Transports, seiner Handhabung und seiner Einbringung an den Setzplatz in der Wachstums- und Vermehrungsstation 100. Das Lagerbehältnis 6 ist, insbesondere nach ihrer Einbringung in den Wachsraum der Wachstums- und Vermehrungsstation 100, sterilisierbar, sei es mit Hilfe von Flüssigkeiten, Strahlung, Gasen oder einer Mischung der vorgenannten Varianten. Das Lagerbehältnis 1 schützt das Entwicklungsgut 6 während dieses Sterilisationsprozesses.

Nach der Sterilisierung kann das Lagerbehältnis 1 durch ein automatisier- und/oder kontrollierbares Öffnungsverfahren geöffnet werden.

Varianten dieses Öffnungsverfahrens umfasst
I. mechanische Verfahren wie Platzen, Sprengen, Schneiden, Auflösen, Durchdringen, Aufschrauben, (Auf)drehen, Drücken, Schall, Ultraschall, Vibration, Klopfen, Schlagen, Perforieren
II. und/oder durch Degradation bzw. Zersetzung insbesondere auf thermischem und/oder chemischem Weg
III. und/oder durch Fermentierung
IV.und/oder durch Energieeintrag, wie z.B. Gleichstrom, Wechselstrom, elektromagnetische Strahlung, Magnetismus, Laser
V. und/oder eine Mischung davon aus zwei oder mehr der vorgenannten Verfahren

Das Lagerbehältnis 1 enthält, wie bereits zuvor beschrieben, Substanzen für die Versorgung des Entwicklungsgutes 6 während der Lagerung, des Transportes und des Einbringens in die Wachstums- und/oder Vermehrungsstation 100. Hierzu können u.a. Wasser, Nährstoffe und andere Medien oder Substanzen gehören. Die Substanzen können insbesondere den Entwicklungszyklus entweder unterbrechen, hemmen, verlangsamen, fördern, beschleunigen und/oder fortführen.

Die Wandung des Lagerbehältnisses 1 kann zudem unter Einhaltung der Sterilität des Entwicklungsgutes 6 einen Gasaustausch erlauben. Vorzugsweise kann die Wandung des Lagerbehältnisses alternativ oder zusätzlich zur Diffusionssperrschicht zumindest ein Wandungssegment vorgesehen sein, welches die Gasdiffusion erlaubt, jedoch diffusionsdicht gegenüber Flüssigkeiten ist. Besonders bevorzugt kann eine Membran in die Wandung eingearbeitet sein, welche einen einseitigen Austritt von Gas, allerdings nicht von Flüssigkeiten erlaubt. Insbesondere kann eine Membran in die Wandung eingearbeitet sein, die gezielt spezifische Gase oder Gasgemische austreten resp. eintreten lässt.

Außenseitig kann das Lagerbehältnis 1 eine Markierung aufweisen, welche eine Identifizierung des Entwicklungsgutes 6 und die Position bzw. den Setzplatz des Lagerbehältnisses 1, sowie ggf. weitere Informationen aufweist. Die Markierung und folglich mit ihr das Entwicklungsgut 7, kann vorzugsweise als QR-Code, Barcode, Datamatrix code, Punkt- bzw. dot-matrix, Symbole, Farbe oder Farben, Muster, RFID oder eine Mischung dieser obgenannten Varianten ausgebildet sein, so dass das Entwicklungsgut 7 identifizierbar und rückverfolgbar ist.

Die Erfindung betrifft weiter ein Kultivierungssystem, bestehend aus mindestens einer Wachstums- und/oder Vermehrungsstation 100 oder aus mehreren zusammengefügten, modularen Wachsräumen, im Folgenden Farm 200 genannt, in welchem der Prozessschritt III stattfindet

Eine jede einzelne Wachstums- und/oder Vermehrungsstation 100 kann als ein Container mit Überseecontainer-Normmaßen, also einen Großraumbehälter gemäß ISO 668 in der aktuellen Fassung zum Prioritätszeitpunkt der vorliegenden Erfindung, ausgebildet sein. Dadurch ist die Modularität und Stapelfähigkeit einzelner Module gegeben

Der Container kann insbesondere per Lastwagen oder Schiff transportiert werden, ist mittels üblicher Vorrichtungen nach ISO 668 stapel- und/oder verbolzbar und somit ein Zusammenschließen von mehreren Wachstums- und/oder Vermehrungsstationen 100 und ggf. von weiteren Stationen zu einer Farm 200 möglich wird.

Die Wachstums- und/oder Vermehrungsstation 100 ist gegenüber der Außenwelt thermisch durch eine innere Isolation 15 getrennt und kann ein gewünschtes Klima bereitstellen. Insbesondere ist die innere Isolation 15 aus Polystyrol, Polyurethan, Cellulose, mineralischen Stoffen, Glas resp. Schäumen, Fasern oder Wollen dieser hergestellt. Somit wird das Entwicklungsgut 6 eine eigene Hemisphäre geboten, die im weiteren Innenatmosphäre genannt wird, welche für jede Wachstums- und/oder Vermehrungsstation 100 oder für die ganze Farm entsprechend der durch das Entwicklungsgut 6 geforderten Bedingungen dem Entwicklungsgut 6 über die diversen Reifezyklen zur Verfügung stellen kann.

Die Wachstums- und/oder Vermehrungsstation 100 weist zudem eine schleusenähnliche Installation und/oder Andockstelle 16 und optional in der Erweiterung zu einem Verarbeitungssystem eine Unterhaltseinheit 17 an einer ersten Stirnseite der Wachstums- und/oder Vermehrungsstation 100 auf. Durch die Unterhaltseinheit 17, im Weiteren Maintenance-Einheit 300 genannt, kann der Unterhalt der Wachstums- und/oder Vermehrungsstation 100 und/oder die Ernte kontaminationsfrei und steril erfolgen.

Dabei kann die Maintenance-Einheit 300 u.a. eine Doppeltür 18 aufweisen, welche die schleusenähnliche Installation ermöglicht. Die Ernte erfolgt dabei in Prozessschritt IV.

Während der Prozessschritte II-IV sollte die Wachstums- und/oder Vermehrungsstation 100 nicht durch Menschen betreten werden. Dies ist notwendig um dem Entwicklungsgut 6 eine aseptische Innenatmosphäre zu bieten, und in Konsequenz auf Pestizide zur Behandlung des Entwicklungsgutes 6 verzichtet werden kann.

Eine Medienversorgung kann über eine zweite Stirnseite, der Technikseite 19, der vorzugsweise als Container ausgebildeten Wachstums- und/oder Vermehrungsstation 100 erfolgen und somit für Wartungs- und Unterhaltsarbeiten von außen, insbesondere auch während des Prozessschrittes III zugänglich sein. Sämtliche für das Wachstum und die Vermehrung des Entwicklungsgutes 6 benötigten Materialien und Medien können jeweils über die Technikseite 19 der Wachstums- und/oder Vermehrungsstation 100 zu- und/oder abgeführt werden. Optional und besonders bevorzugt können für dieses Zu- und/oder Abführen Maintenance-Zyklen vorgesehen sein, um die Kontrolle und Aseptik innerhalb des Wachsraumes aufrecht zu erhalten.

Die Wachstums- und/oder Vermehrungsstation 100 weist mindestens eine eigene Energieversorgungs- und/oder -verwaltungseinheit 20 auf. Die Energieversorgungs- und/oder -verwaltungseinheit 20 weist mindestens ein eigenes Kommunikationsmodul 21 auf. Um für das Entwicklungsgut 6 wichtige Prozesse permanent zur Verfügung zu stellen, kann die Energieversorgungs- und/oder -verwaltungseinheit 20 zudem über eine unterbruchsfreie Stromversorgung 22 verfügen.

Als Energieversorgungs- und/oder -verwaltungseinheit kann auch lediglich ein Stromanschluss für eine Fremdquelle vorgesehen sein oder aber ein Stromzwischenspeicher, z.B. ein Notstromaggregat, oder aber eine Stromüberbrückung z.B. als USV.

Um eine aseptische Produktion des Entwicklungsgutes 6 zu erlauben, in welchem das Entwicklungsgut 6 von der Außenwelt komplett hermetisch getrennt ist, sollte die technische Auslegung der Wachstums- und/oder Vermehrungsstation 100 derart erfolgen, dass für den Prozessschritt II bis IV Reinraumkriterien nach ISO und/oder GMP für die biologische Kontamination innerhalb des Wachsraumes bestehen.

Weiterhin kann eine Lichtquelle 23 vorgesehen sein, um dem Entwicklungsgut 6 kalibrierbare Lichtverhältnisse zu bieten, also dem Entwicklungsgut 6 auch alle sonstigen atmosphärischen Bedingungen wie etwa den Tag/Nacht-Zyklus oder ein gefordertes veränderliches Lichtspektrum und Lichtintensität zur Verfügung zu stellen. Insbesondere kann dem Entwicklungsgut 6 eine konstante Lichtstärke während des gesamten Wachstumszyklus zur Verfügung gestellt werden.

Weiterhin weist die Wachstums- und/oder Vermehrungsstation 100 eine Befeuchtungs- und/oder eine Ventilationseinheit, nachstehend Klimagerät 24 genannt auf, um dem Entwicklungsgut über vorzugsweise Ventilationsschlitze 25 die benötigte Luftfeuchtigkeit, die atmosphärische Zusammensetzung und Winde zur Verfügung zu stellen.

Weiterhin weist die Wachstums- und/oder Vermehrungsstation 100 ein Nährstoffresp. Bewässerungssystem 26 auf, um dem Entwicklungsgut 6 die benötigten abgestimmten Nährstoffe für den gesamten Wachstumszyklus bereitzustellen und an den vom Entwicklungsgut 6 geforderten Stellen, etwa am Wurzel- oder Blattwerk, zur Verfügung zu stellen. Insbesondere ist es vorteilhaft die Nährstoffe in einem Bewässerungssystem 26 so zuzuführen, dass die Bestandteile resp. die Zusammensetzung des Nährmediums bekannt ist. Dies kann dadurch realisiert werden, dass Wasser durch eine Wasseraufbereitung 27 desinfiziert, entionisiert und/oder von Schwermetallen befreit wird. Dieses aufbereitete Wasser kann in einem Reservoir 28 zwischengelagert werden resp. dahingehend recycled werden, um dem Nährstoffmedium resp. dem Bewässerungssystem 26 wieder zugeführt zu werden.

Eine aeroponische Kultivierung zeichnet sich dadurch aus, dass Wurzeln mit Aerosol einer Lösung aus Nährstoffen und Wasser benetzt werden. Zur Realisierung dieser Technologie kann die Wachstums- und/oder Vermehrungsstation 100 beispielsweise ein Niedrigdrucksystem aufweisen. Alternativ ist auch ein Hochdrucksystem oder ein Ultraschallzerstäuber einsetzbar. Im bevorzugten Fall eines Niederdrucksystems kann eine Vernebelung der Lösung mittels Piezoelementen erfolgen.

Alternativ oder zusätzlich kann auch eine hydroponische Kultivierung mittels NFT (nutrient film technique) ermöglicht werden. Dabei wird das Entwicklungsgut in Netztöpfen oder dergleichen eingesetzt, welche es ermöglichen, dass Wurzel bei Pflanzen oder Myzel bei Pilzen oder andere Teile des Entwicklungsgutes aus dem Topf herausragen und in eine flüssigkeitsführende Rinne hineinragen, in welcher eine Nährstofflösung geführt ist. Diese Rinne ist leicht schräg und gibt somit eine Strömungsrichtung für die Nährstofflösung vor. Die Nährstofflösung kann sodann über Abführöffnungen abgeführt und in einem Sammelbereich unterhalb der Rinne gesammelt und ggf. im Kreislauf in die Rinne rückgeführt, vorzugsweise gepumpt, werden. Zwischen der Nährstofflösung und dem Topf ist ein Luftraum angeordnet, welcher durch die Teile des Entwicklungsgutes überwunden bzw. überbrückt werden muss, um an die Nährstofflösung zu gelangen.

Das Nährstoffmedium wird aus Wasser, das zuvor die Wasseraufbereitung 27 passiert hat und/oder aus dem Reservoir 28 mittels Aktuatoren 29 und/oder gravitativ gefördert wurde und den verschiedenen Nährstoffen, die vorteilhafterweise in Nährstoffbehältern 30 gelagert sind, angemischt. Vorteilshafterweise werden die Nährstoffe aus dem Nährstoffbehältern 30 durch eine Mischkammer 31 passiv und/oder aktiv gemischt. Dadurch lassen sich Gradienten, Pulse, Stufen oder Wechsel von Nährstoffmedienzusammensetzungen realisieren, die abgestimmt auf den Wachstumszyklus des Entwicklungsgutes 6 die Entwicklung des Entwicklungsgutes 6 beeinflussen lassen.

Zudem weist die Wachstums- und/oder Vermehrungsstation 100 einen geschlossenen Regelkreis mit einer Steuer- und/oder Auswerteeinheit 32 und mehreren Aktuatoren 29 und Sensoren 33 auf. Der Regelkreis detektiert die Konzentrationen der Stoffe sowohl in der Innenatmosphäre als auch in der zugeführten Nährmedien, und kann Veränderungen der Innenatmosphäre analysieren. Sodann ermöglicht die Steuer- und/oder Auswerteeinheit 32 durch Steuerung und/oder Regelung der Aktuatoren 29 des Regelkreises, dass dem Entwicklungsgut 6 aufgrund der analysierten Daten eine angepasste Innenatmosphäre und/oder Nährmedien zur Verfügung gestellt wird. Dies kann zeitnah bzw. just in time erfolgen, so dass der bevorzugte Zeitraum zwischen der Messung und der Anpassung weniger als 30 min, vorzugsweise weniger als 15 min, beträgt.

Vorteilhaft im Rahmen der vorliegenden Erfindung ist der vorgenannte Regelkreis als Teil des Wachstums- und/oder Vermehrungsstation 100. Der Regelkreis kann vorteilhaft als geschlossener Regelkreis ausgebildet sein. Er kann eine Steuer- und/oder Auswerteeinheit 32 zu dessen Steuerung aufweisen. Die Steuerung erfolgt insbesondere nach der Auswertung von Sensorsignale durch entsprechende Sensoren 33 innerhalb der Wachstums- und/oder Vermehrungsstation 100. Die Steuer- und/oder Auswerteeinheit 32 und vorzugsweise auch der gesamte Regelkreis ist vorzugsweise fernbedienbar und besonders bevorzugt von einem externen Gerät über ein Kommunikationsmodul 21 bidirektional ansteuerbar. Eine komplexere Analyse von Messdaten kann durch das externe Gerät erfolgen. Die Datenspeicherung kann insbesondere durch das externe Gerät erfolgen. Der Begriff "externes Gerät" umfasst dabei auch eine IT-Infrastruktur, wie beispielsweise eine Cloud oder eines neuronalen Netzwerkes.

Zwischen dem Regelkreis und dem externen Gerät kann eine Datenübertragung, vorzugsweise eine bidirektionale Datenübertragung, erfolgen. Zum Ansteuern des Regelkreises kann zudem auch eine IT-Infrastruktur in Form des künstlichen neuronalen Netzwerkes genutzt werden, welches z.B. Tendenzen aus den Sensordaten erkennt und durch entsprechende Analyselogarithmen Schlussfolgerungen über den Gesamtzustand des Entwicklungsgutes 6 ziehen kann. An dieser Stelle kann somit künstlich generierte Schwarmintelligenz zur Auswertung einer Vielzahl von Sensordaten eingesetzt werden. Die Datenübertragung zwischen dem Regelkreis und dem externen Gerät erfolgt bevorzugt über ein Kommunikationsmodul 21, dass über GSM, WiFi, LoRa und/oder Bluetooth sendet und/oder empfängt.

Der Regelkreis zeichnet sich dadurch aus, dass die erhobenen Daten der Sensoren 33 geloggt werden und durch eine dem Entwicklungsgut 6 entsprechende Auswertung herangezogen werden, um Bedingungen zu halten, wiederherzustellen und/oder herbeizuführen. Diese Bedingungen können dabei vorteilhafterweise sein, dass eine gewünschte oder benötigte Innenatmosphäre oder eine Veränderung der Nährstoffzusammensetzung angesteuert wird. Das kann sowohl in Echtzeit als auch in einem zeitversetzten bzw. einem sogenannten time-shift Verfahren angewendet werden. Der Regelkreis kann Daten aller Wachsräume und aller Farmen 200 für die Auswertung heranziehen, sowohl simultan als auch über eine Zeitspanne. Die Daten können zum Zweck einer Analyse der Wachstumsphasen, des Gesundheitszustandes oder der Maximierung und/oder Minimierung von Parametern des Entwicklungsgutes 6, insbesondere zur Ermittlung von Grenz- und/oder Sollwerten, herangezogen werden.

Zur Analyse können zudem externe Literatur, Analysen oder andere Daten ergänzt, verifiziert oder plausibilisiert werden. Die Daten können durch Verfahren der mathematischen Analyse, Interpretation, Kausalität, Sinnbildung, Entscheidungsfindung und/oder Prognose herangezogen werden. Die Analysen können durch statistische Methoden verfeinert, ergänzt, erweitert und/oder komplettiert werden. Insbesondere bieten sich statistische Methoden, wie Fehleranalyse, ANOVA, Reihenentwicklungen, Maxima, Minima, Differential, Integral und/oder Konvergenzanalysen an.

Die Analyse, die Verwaltung, die Speicherung, die Archivierung und die Sicherung der Daten kann von der Wachstums- und/oder Vermehrungsstation 100 oder von der Farm 200 entfernt in dem externen Gerät erfolgen und bedient sich gängiger Übermittlungsprotokolle, Techniken und Infrastruktur, im weiteren Datenverarbeitung genannt.

Dabei zeichnet sich die Datenverarbeitung dadurch aus, dass eine bidirektionale Kommunikation mit der Wachstums- und/oder Vermehrungsstation 100 und/oder der Farm 200 möglich ist und dass die Daten durch Algorithmen mittels KI (künstliche Intelligenz, machine learnings oder des supervised machine learnings (zentral) verwertet, verarbeitet und die daraus gewonnenen Erkenntnisse für die Kultivierung des Entwicklungsgutes 6 verwendet werden.

Weiter weist die Wachstums- und/oder Vermehrungsstation 100, wie bereits zuvor erwähnt, Lagergestelle 14 bzw. Regale zur Lagerung der Lagerbehältnisse 1, insbesondere in der Ausgestaltung als Lagerbehältnis, auf. Das Lagergestell 14 kann innerhalb des Wachsraumes verfahrbar angeordnet sein. Optional kann das jeweilige Lagergestell 14 auch in eine angeschlossene Maintenance-Einheit 300 verfahrbar sein. Eine vorteilhafte Variante einer solchen Lagerung kann eine Rollenlagerung sein, wobei die Rollen auf einer Führung, z.B. einer Führungsschiene am Boden oder an der Decke der Wachstums- und/oder Vermehrungsstation 100, geführt sind.

Zur Reinigung und/oder für das neue Beschicken der Lagergestellen 14 mit Tablaren 34 und/oder Lagerbehältnisse 1 kann ein jeweiliges Lagergestell 14 aus dem jeweiligen Wachsraum ausgefahren werden.

Die Lagergestelle 14 sind dabei vorzugsweise Regalsysteme, die alle zur Versorgung des Entwicklungsgutes 6 mit Medien notwendigen Anschlüsse und/oder Installationen enthalten. Diese Medien sind vorzugsweise Nährstoffe, Wasser, Gase, Wind und/oder ggf. weitere wachstums- oder vermehrungsbeeinflussende Substanzen. Notwendige Installationen und/oder Anschlüsse können vorzugsweise setzplatzindividuell ausgebildet sein und können z.B. Sprühdüsen, Zu- und/oder Ableitungen, Verteilungssysteme, elektrische Signalleitungen, Stromzuleitungen, setzplatzindividuelle Funkmodule, sowie setzplatzindividuelle Sensoren 33 und/oder Aktuatoren 29 umfassen. Nährstoffbehälter 30 für Nährstoffe und andere Medien können setzplatzindividuell oder Lagergestell-individuell vorgesehen sein, was besonders von Vorteil ist, sofern unterschiedliches Entwicklungsgut 6, z.B. verschiedene Pflanzensorten, in unterschiedlichen Setzplätzen oder Lagergestellen angeordnet ist.

Ein jeweiliges Lagergestell 14 kann mehrteilig aufgebaut sein und vorzugsweise einlegbare Tablare 34 und/oder Auffangwannen aufweisen mit einer oder mehreren Aufnahmeöffnungen für ein oder mehrere Lagerbehältnisse 1. Die Tablare 34 können dabei gesondert sterilisiert oder ggf. als single-use Elemente auch verworfen und bei erneuter Benutzung gegen neue Tablare 34 ausgetauscht werden.

Sofern lediglich eine einzige Sorte an Entwicklungsgut 6 in einer Wachstums- und/oder Vermehrungsstation 100 vorliegt, kann auch ein einziges Reservoir 28 und/oder Nährstoffbehälter 30 pro Wachstums- und/oder Vermehrungsstation 100 oder sogar lediglich pro Verarbeitungssystem 400 vorgesehen sein.

Die Lagergestelle 14 können zudem zur Entsorgung der verbrauchten Medien notwendigen Installationen enthalten. Die vorhergehenden Ausführungen zu dem Reservoir 28 und Nährstoffbehälter 30 sind entsprechend auch für die Auffangtanks für Restmedium anzuwenden.

Die Tablare 34 sind vorteilhafterweise modular aufgebaut, wobei das Entwicklungsgut 6 je Tablar 34 vorzugsweise neun oder mehr Setzplätze für das sichere und kontrollierte Halten des Entwicklungsgutes 6 während der Kultivierung bereitgestellt werden können. Die Tablare 34 ermöglichen weiter eine effiziente Ernte, da vorzugsweise eine Auffangvorrichtung für die Wurzeln oder den Nährstoffmedium zugewandter Teil des Entwicklungsgutes 6 am Tablar 34 befestigt ist. Dies kann z.B. ein Netz, ein Beutel oder/und eine Wanne sein.

Die verfahrbaren Lagergestelle 14 sind so verbaut, dass innerhalb der Innenatmosphäre keine Verkehrsflächen notwendig sind und so der Wachsraum zur Lagerung des Entwicklungsguts 6 maximal genutzt wird.

Mehrere Wachstums- und/oder Vermehrungsstationen 100 können zu größeren Einheiten, sogenannten Farmen 200, zusammengeschlossen werden. Farmen 200, also größere Anlagen aus mehreren, beliebig vielen einzelnen Wachsräume, können zu deren Unterhalt, Ernte und Versorgung über eine einzige Maintenance-Einheit 300, z.B. einen gesonderten Container, betrieben werden.

Es ist jedoch auch möglich lediglich aus einem eine Wachstums- und/oder Vermehrungsstation 100 vorzusehen und die Maintenance-Einheit 300 auch angrenzend an den Wachstums- und/oder Vermehrungsstation 100 innerhalb eines einzigen Containers, vorzugsweise benachbart zum Wachsraum, anzuordnen.

Sowohl die Maintenance-Einheit 300, die Prozess-Einheit 500 als auch die Wachstums- und/oder Vermehrungsstation 100 bilden somit ein Verarbeitungssystem 400, welches ein Wachstum/Vermehrung des Entwicklungsgutes 6 als auch die Ernte, die Prozessierung und die Verpackung des geernteten Entwicklungsgutes 6 unter aseptischen und insbesondere hermetischen Bedingungen ab dem Zeitpunkt des Einsetzens und des Verschlusses des Entwicklungsgutes 6, z.B. als Samen im Lagerbehältnis 1 gewährleistet. Dabei wird das Verarbeitungssystem auf einen oder mehrere Großraumbehälter bzw. Container, verteilt, welche das Aufrechterhalten einer hermetischen Innenatmosphäre zumindest für das Wachstum und die Vermehrung, jedoch bevorzugt auch für die Ernte, Prozessierung in einer Prozess-Einheit 500 und/oder Verpackung gewährleisten.

So können beispielsweise Heilpflanzen oder dergleichen unter sterilen Bedingungen gezüchtet werden. Das komplette Verarbeitungssystem 400 oder zumindest die Wachstums- und Vermehrungsstation 100 und besonders bevorzugt jeder Container des Verarbeitungssystems 400, weist dabei Zu- und/oder Ableitungen für die Einleitung eines Reinigungsmediums, vorzugsweise eines CIP-Mediums, z.B. Dampf oder dergleichen, auf - damit das System nach dem Gebrauch für eine erneute Benutzung sterilisiert ist.

Die Maintenance-Einheit 300 erlaubt die Manipulation am Entwicklungsgut 6 und die Überwachung des Entwicklungsgutes 6 mittels automatisierter Systeme, etwa Robotersystemen und/oder eine zeitversetzte Ernte des Entwicklungsguts 6 und erlaubt so eine Manipulation von außen ohne die aseptische Innenatmosphäre zu kontaminieren. Hierbei wird auf einem Datenspeicher 35 einer Steuer- und Auswerteeinheit 32 einer oder mehrere Datensätze zur Art des Entwicklungsgutes 6 und dem gewünschten Wachstumsstadium (z.B. Pflanzengröße, Blattgröße, Fruchtgröße, Reifegrat einer Frucht, Pilzgröße, Bakterienzahl, usw.) für die Initiierung eines Ernteprozesses hinterlegt. Wird ein Sollwert oder die Kombination mehrerer des Wachstumsstadiums entsprechenden Sollwerten erreicht, so kann die Steuer- und/oder Auswerteeinrichtung 32 den Ernteprozess initiieren.

Dies kann u.a. die Aktivierung einer Erntemaschine, insbesondere eines Ernteroboters, umfassen oder ggf. auch zunächst die hermetische Kopplung einer Maintenance-Einheit 300 an die Wachstums- und/oder Vermehrungsstation 100, sowie das anschließende Verfahren der Erntemaschine, insbesondere des Ernteroboters, in die Wachstums- und/oder Vermehrungsstation 100 oder alternativ ein Verfahren eines Lagergestells 14 aus der Wachstums- und/oder Vermehrungsstation 100 in die angeschlossene Maintenance-Einheit 300.

Alternativ kann die Öffnungsvorrichtung auch lediglich in der Wachstums- und/oder Vermehrungsstation 100, z.B. als Teil eines verfahrbaren Roboters angeordnet sein, welcher keinem Lagergestell 14 zugeordnet ist. Diese Lösung ist allerdings weniger bevorzugt, da sich der Zeitaufwand zum Öffnen jedes Lagerbehältnisses bei dieser Lösung erhöht.

Die Maintenance-Einheit 300 kann Lager- oder Transportelemente, z.B. Lagerrollen, zum vertikalen und lateralen Manövrieren aufweisen. Beispielsweise kann ein Hebemechanismen, insbesondere ein Hebemechanismus, und/oder eine Mecanum-Antriebsvorrichtungen, z.B. Mecanum-Räder, als Teil der Maintenance-Einheit vorgesehen sein. Eine einzelne Maintenance-Einheit 300 kann viele Wachsräume, Prozess-Einheiten 500 und/oder Farm 200 bedienen.

Die Maintenance-Einheit 300 kann zum Unterhalt einer einzelnen Wachstums- und/oder Vermehrungsstation 100. Prozess-Einheit 500 oder einer Farm 200 verwendet werden. Die Maintenance-Einheit 300 kann das Entwicklungsgut 6 ernten und prozessieren, so zum Beispiel trocknen, zerkleinern, separieren, temperieren, verpacken, destillieren, extrahieren, reinigen, fotografieren und/oder analysieren. Einer oder mehrere dieser Schritte können einzeln oder in Kombination im Prozessschritt III durchgeführt werden. Hierfür kann die Maintenance-Einheit 300, also der Container jeweils oder zusammen eine Erntemaschine, eine Trocknungsvorrichtung, eine Zerkleinerungsvorrichtung, z.B. ein Häcksler und/oder eine Mühle, eine Filtrations- und/oder Zentrifugiereinrichtung, eine Temperiervorrichtung, z.B. eine Schockfrostanlage, eine Verpackungsvorrichtung, eine Destille, eine Extraktionsvorrichtung, ein Reinigungsmodul, ein Foto- oder Videoapparat und/oder ein Analysengerät, vorzugsweise ein HPLC, GC (Gaschromatigraphie), UV-Vis, Raman und/oder IR-Analysengerät aufweisen. Neuere Geräte, z.B. Prozessphotometer, ermöglichen zudem eine Inline-Messung im Verarbeitungsprozess und können in vorliegenden Fall angewandt werden.

Vorteilhafterweise werden die oben genannten Prozesse in einen eigenen Container, i.e. Prozess-Einheit 500 ausgelagert und die Maintenance-Einheit 300 beliefert die Prozess-Einheit 500 mit geerntetem Entwicklungsgut 6 aus der Wachstums- und Vermehrungsstation 100 über die jeweilige Andockstellen 16 an der Wachstums- und Vermehrungsstation 100, der Maintenance-Einheit 300 und der Prozess-Einheit 500, um eine durchgehende, kontaminationsfreie Prozessierung eins Entwicklungsgutes 6 zu gewährleisten.

Die Maintenance-Einheit 300 kann den atmosphärischen Bedingungen und Bedürfnissen der Wachstums- und/oder Vermehrungsstation 100 für die Durchführung des Prozessschrittes III und/oder IV angepasst werden. Weiter kann die Maintenance-Einheit 300 eine Personenschleuse aufweisen, um Personal unter Reinraumbedingungen oder Reinraum-ähnlichen Bedingungen ein- und auszubringen oder um das Andocken weiterer Einheiten, e.g. eine Prozess-Einheit 500, zu erleichtern. Der Prozessschritt III und/oder IV in der Maintenance-Einheit 300 kann manuell, semi-manuell oder automatisiert durchgeführt werden. Durch die gleichbleibenden atmosphärischen und/oder mikrobiellen Bedingungen bei der Herstellung des Entwicklungsgutes 6 bis zum Prozessschritt IV kann eine im hohen Maße gleichbleibende Qualität und Homogenität des erntereifen Entwicklungsgutes 6 und dessen Prozessierung erreicht werden. Die Maintenance-Einheit 300 und/oder die Prozess-Einheit 500 kann einen Lagerraum für nicht prozessierte und/oder prozessierte Güter aufweisen, der temperiert, atmosphärisch kontrolliert und/oder unter Schutzgas gesetzt werden kann, um das Entwicklungsgut 6 vor ungewünschten Einflüssen der Umwelt außerhalb des Containers zu schützen. Die Lagerbewirtschaftung kann durch Lifte, PaterNoster oder ähnliche System automatisiert oder manuell sein.

Nachfolgend wird detaillierter auf die in den Figuren dargestellten Ausführungsvarianten eingegangen. In Fig. 1 ist ein Lagerbehältnis 1 in Form einer Kapsel dargestellt. Das Lagerbehältnis 1 weist eine Wandung 7 auf und hat ein Mittelsegment 2 in Form eines röhrenartigen Bereichs, ein als obere Kappe ausgebildetes erstes Endsegment 8 und ein als untere Kappe ausgebildetes zweites Endsegment 8. Die obere Kappe bzw. das erste Endsegment kann dabei gaspermeabel ausgebildet sein. Im röhrenartigen Bereich, i.e. dem Mittelsegment 2, gibt es eine Matrix bzw. ein Substrat 4 mit Nährstoffen 5 in der das Entwicklungsgut 6 angeordnet ist. Zwischen dem Mittelsegment 2 und den Endsegmenten 8 sind jeweils mechanische Schnittstellen und/oder Sollbruchstellen 9 ausgebildet. Die mechanische Schnittstelle 9 kann z.B. einen Rastmechanismus umfassen. Weiter ist zumindest ein Begrenzungsanschlag 13 als Haltevorrichtung umfangsverteilt an der Wandung 7 vorgesehen. Umfangsverteilt bedeutet dabei mehrere auf einer Höhe des Mittelsegments um den Umfang verteilte Einzelsegmente oder ein einziges vollumfänglich auf einer Höhe verlaufendes Segment, welches einen Axialanschlag darstellt und die Einstecktiefe des Mittelsegments 2 in einer Öffnung begrenzt.

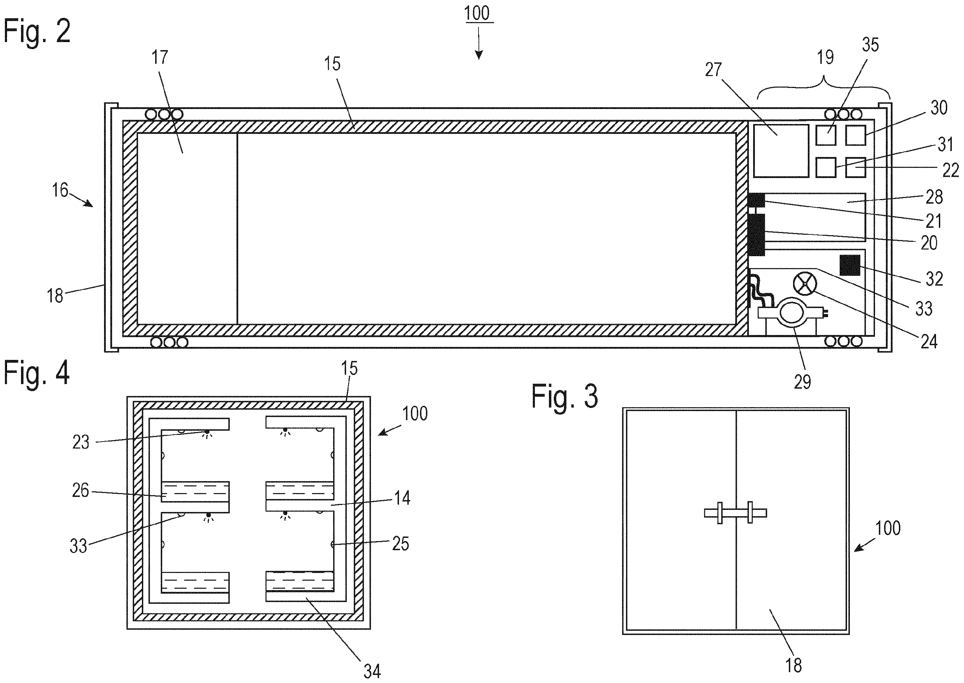

Fig. 2-4 zeigt eine als Container ausgebildete Wachstums- und Vermehrungsstation 100. Diese hat eine Außenhülle mit Vorrichtungen, die eine Kraftschlüssige Verbindung zu anderen Containern oder Tragvorrichtungen nach ISO 668 zulässt, eine innere Isolation 15, eine Versorgungseinheit 20 und eine Andockstelle 16 mit Doppeltüre 18. Innerhalb des Containers kann es eine Anordnung aus einem oder vorzugsweise mehreren Lagergestellen 14 bzw. ein Regalsystem geben, welches Sensoren 33, ein Bewässerungssystem 26, eine Belüftungsanlage oder zumindest Teile davon, wie z.B. Ventilationsschlitze 25, und die Lichtquelle 23 beinhaltet. Die Versorgungseinheit 20 kann jeweils eine oder mehrere Aktuatoren 29, wie Pumpen und/oder Ventile, Sensoren 33, eine Steuer- und Auswerteeinheit 32, ein Klimagerät 24, eine Wasseraufbereitung 27, ein Reservoir 28, Nährstoffbehälter 30, Mischkammern 31, eine Energieversorgungs- und -verwaltungeinheit 20 und ein Kommunikationsmodul 21, vorzugsweise in der Ausgestaltung als Funkmodul, beinhalten. Der Container kann zu einer Farm 200 zusammengeschlossen werden.

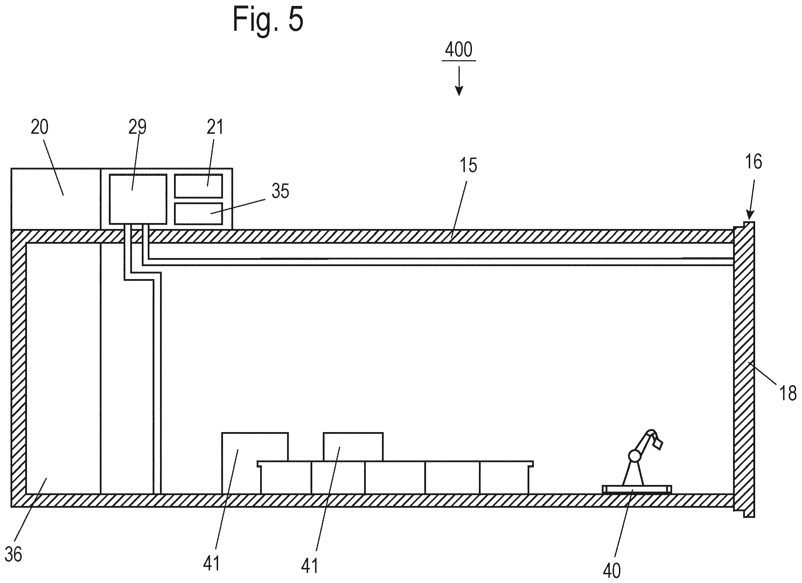

Fig. 5 zeigt eine Maintenance-Einheit 300. Diese kann einen Hebemechanismus 37 und/oder einen Antrieb 38 aufweisen um vertikal und horizontal verfahrbar zu sein. Die Maintenance-Einheit 300 kann an einer Stelle, insbesondere an einer ersten Stirnseite, eine Andockstelle 16 aufweisen und an anderer Stelle, insbesondere an einer zweiten Stirnseite, eine Personenschleuse 34, und eine Energieversorgungs- und -verwaltungeinheit 20 sowie innenliegend eine Isolation 15. Der Innenraum kann ein Fördersystem 37 aufweisen um das Lagergestell 14 zu fördern, sowie eine roboterähnliche Installation 40 zur automatisierten Ernte. Zusätzlich kann die Maintenance-Einheit 300 einen Lagerraum 41 aufweisen.

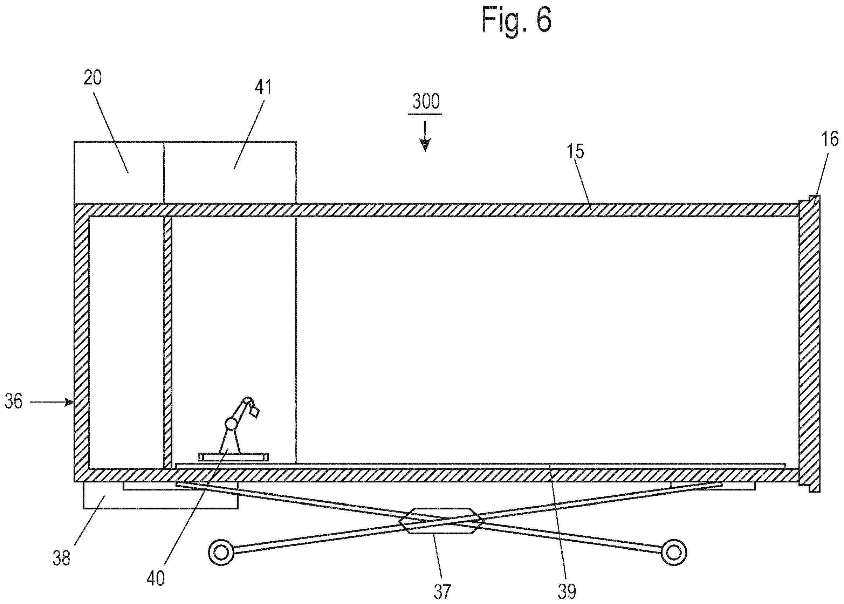

Fig. 6 zeigt eine Prozess-Einheit 500. Diese kann eine Energieversorgungs- und - verwaltungseinheit 20, eine Personenschleuse 39, eine Andockstelle 16 und eine Innenisolation 15 aufweisen. Die Energieversorgungs- und -verwaltungseinheit 20 kann eine Technikseite 19, ein Kommunikationsmodul 21 und/oder ein Reservoir 28 aufweisen. Die Andockstelle 16 kann eine Doppeltür 18 aufweisen. Im Innenraum der Prozess-Einheit 500 kann ein oder mehrere technische Installationen, mehrheitlich Roboterähnliche Installationen 40 installiert sein und ein Lagerbereich 41 für unprozessierte und prozessierte Güter sein.

### Bezugszeichen

- 1: Lagerbehältnis
- 2: Mittelsegment
- 3: Innenraum
- 4: Substrat
- 5: Nährstoffen
- 6: Entwicklungsgut
- 7: Wandung
- 8: Endsegment
- 9: Mechanische Schnittstelle
- 10: Schutzschicht
- 11: Sperrschicht
- 12: Stützschichten
- 13: Begrenzungsanschlag
- 14: Lagergestell
- 15: Isolation
- 16: Andockstelle
- 17: Unterhaltseinheit
- 18: Doppeltür
- 19: Technikseite
- 20: Energieversorgungs- und -verwaltungseinheit
- 21: Kommunikationsmodul
- 22: Unterbruchsfreie Stromversorgung
- 23: Lichtquelle
- 24: Klimagerät
- 25: Ventilationsschlitze
- 26: Bewässerungssystem
- 27: Wasseraufbereitung
- 28: Reservoir
- 29: Aktuatoren
- 30: Nährstoffbehälter
- 31: Mischkammer
- 32: Steuer- und Auswerteeinheit
- 33: Sensoren
- 34: Tablare
- 35: Datenspeicher
- 36: Personenschleuse
- 37: Hebemechanismus
- 38: Antrieb
- 39: Fördersystem
- 40: Roboterähnliche Installation
- 41: Lagerraum

- 100: Wachstums- oder Vermehrungsstation
- 200: Farm
- 300: Maintenance-Einheit
- 400: Verarbeitungssystem
- 500: Prozess-Einheit

## Patentansprüche

1. Lagerbehältnis (1) umfassend einen Innenraum (3) und ein darin angeordnetes Substrat (4), welches ortsfest an der Wandung des Lagerbehältnisses angeordnet ist, wobei das Lagerbehältnis (1) zudem ein im oder am Substrat (4) angeordnetes biologisches lebendes Entwicklungsgut (6) in Form von Pflanzen, Pilzen und/oder Algen aufweist, **dadurch gekennzeichnet, dass** das Lagerbehältnis (1) als eine zumindest flüssigkeitsdicht- oder feststoffdicht-abgeschlossene Kapsel zur Gewährleistung einer aseptischen Atmosphäre, insbesondere eine Aseptik nach ISO 11135, ISO 11137, ISO 17665-1, ISO 13408-1, EU GMP Annex 1 oder US cGMP mit einem Sterilisationsprozessziel eines SAL (sterility assurance level) von mindestens 10⁻⁵, ausgebildet ist.

2. Lagerbehältnis nach einem der vorhergehenden Ansprüche, **dadurch gekennzeichnet, dass** das biologische lebende Entwicklungsgut (6) als eine Pflanze, insbesondere als klonierte Pflanze, ausgebildet ist.

3. Lagerbehältnis nach einem der vorhergehenden Ansprüche, **dadurch gekennzeichnet, dass** das Lagerbehältnis (1) zumindest ein Endsegment (8), vorzugsweise zwei Endsegmente (8) und ein rohrförmiges Mittelsegment (2) aufweist, wobei das Substrat (4) und das Entwicklungsgut (6), insbesondere die Pflanze, im Mittelsegment (2) angeordnet ist und wobei zumindest eines der Endsegmente (8) gegenüber dem Mittelsegment (2) eine geringere Wandstärke aufweist oder ein gegenüber dem Mittelsegment (2) ein thermisch- und/oder chemisch-angreifbareres Wandungsmaterial aufweist.

4. Lagerbehältnis nach einem der vorhergehenden Ansprüche, **dadurch gekennzeichnet, dass** zumindest eines der Endsegmente (8) aus einem gaspermeablen Material gebildet ist.

5. Lagerbehältnis nach einem der vorhergehenden Ansprüche, **dadurch gekennzeichnet, dass** eine Wandung (7) des Lagerbehältnisses (1), insbesondere des Mittelsegments (2), zumindest bereichsweise oder vollständig aus einem transparenten Material besteht.

6. Lagerbehältnis nach einem der vorhergehenden Ansprüche, **dadurch gekennzeichnet, dass** das Mittelsegment (2) zumindest einen umfangsverteilten Begrenzungsanschlag (13) oder mehrere umfangsverteile Begrenzungsanschläge oder eine konische Form aufweist.

7. Lagerbehältnis nach einem der vorhergehenden Ansprüche, **dadurch gekennzeichnet, dass** das Substrat (4) ein saugfähiges Material ist mit einer Wasseraufnahmekapazität im trockenen Zustand von zumindest 50 g Wasser/cm³.

8. Lagerbehältnis nach einem der vorhergehenden Ansprüche, **dadurch gekennzeichnet, dass** das Lagerbehältnis (1) eine allumfassende sterilisierbare Oberfläche aus einem peroxid- und/oder radikalstabilen Material, insbesondere einem Ozon-, Peressigsäure- und/oder Wasserstoffperoxid-stabilen Material aufweist, vorzugsweise aus einer Beschichtung oder einer Wandung auf Basis von Metallen, vorzugsweise aus Aluminium und/oder Eisen, von Glas, eines Thermoplastes, wie z.B. Polyamid, Polycarbonat, Polyolefin, Polyacryl, Polymethacryl, halogenierte Ethylene und/oder eines Elastomeren, wie z.B. Kautschuk, halogenierte Elastomere, Silikone gebildet ist.

9. Wachstums- und/oder Vermehrungsstation (100) zur Kultivierung von Entwicklungsgut (6), **dadurch gekennzeichnet, dass** die Wachstums- und/oder Vermehrungsstation (100) als ein Großraumbehälter, insbesondere als ein ISO-Container, ausgebildet ist, wobei der Großraumbehälter eine Vorrichtung zur Erzeugung und Überwachung einer aseptischen Innenatmosphäre aufweist.

10. Wachstums und/oder Vermehrungsstation (100) nach Anspruch 9, **dadurch gekennzeichnet, dass** die Wachstums- und/oder Vermehrungsstation (100) einen Wachsraum aufweist mit einer Zu- und/oder Ableitung eines CIP-Mediums, insbesondere von Ozon.

11. Wachstums und/oder Vermehrungsstation (100) nach Anspruch 9 oder 10, **dadurch gekennzeichnet, dass** der Großraumbehälter zumindest ein Lagergestell (14) zur Anordnung eines oder vorzugsweise einer Mehrzahl von Lagerbehältern (1) gemäß einem der vorhergehenden Ansprüche aufweist.

12. Wachstums- und/oder Vermehrungsstation (100) nach einem der vorhergehenden Ansprüche, **dadurch gekennzeichnet, dass** der Großraumbehälter zumindest eines oder vorzugsweise sämtliche der folgenden Vorrichtungen aufweist:
eine Lichtquelle (23) zur Bestrahlung des Entwicklungsgutes;
ein Aktuator, wie Pumpe (29) zum Medientransport, einschließlich einem nährstoffhaltigen Medium und/oder einem Sterilisationsmedium;
einen Sensor (33) zur Überwachung des Gesundheitszustands des Entwicklungsgutes (6);
eine Steuer- und/oder Auswerteeinheit (32) zur Steuerung eines Regelkreises zur Anpassung der Innenatmosphäre an die vom Sensor (33) erfassten Änderungen auf zumindest einen vorgegebenen Sollwert;
eine Wasserversorgungs-(26) und/oder -aufbereitungseinheit (27);
ein Klimagerät (24);
ein Nährstoffbehälter (30);
eine Energieversorgungs- und/oder -verwaltungseinheit (20);
eine Mischkammer (31) und/oder
ein Kommunikationsmodul (21) zum Datenaustausch mit einem externen Gerät.

13. Kultivierungssystem umfassend eine Wachstums- und/oder Vermehrungsstation (100) sowie eine Maintenance-Einheit (300) und/oder eine Prozess-Einheit (500), insbesondere eine Ernteeinheit, wobei die Maintenance-Einheit mit der Wachstums- und/oder Vermehrungseinheit zur Überführung von Entwicklungsgut oder Maschinen zwischen den beiden Einheiten (100 und 300 oder 500) verbunden ist und wobei die Maintenance-Einheit (300) oder Prozess-Einheit (500) zumindest in Kombination mit der Wachstums- und/oder Vermehrungsstation (100) ausgerüstet ist zum Aufrechterhalten einer aseptischen Innenatmosphäre.

14. Verfahren zur Kultivierung von Entwicklungsgut (6), **gekennzeichnet durch die folgenden Schritte**
a) Bereitstellen eines Lagerbehälters (1) nach einem der vorhergehenden Ansprüche,
b) Einsetzen des Lagerbehälters (1) in ein Lagergestell (14) einer Wachstums- und/oder Vermehrungsstation (100) nach einem der vorhergehenden Ansprüche und zumindest einseitig-endständiges Öffnen des Lagerbehälters (1), vorzugsweise beidseitig-endständiges Öffnen des Lagerbehälters (1)
c) Überwachen des Wachstums und/oder Vermehrung des Entwicklungsgutes (6) in der aseptischen Innenatmosphäre, bis zur Detektion eines Wachstums- und/oder Vermehrungsstadiums für die Ernte;
d) Einsatz der Maintenance-Einheit (300) und/oder Prozess-Einheit (500) unter Aufrechterhaltung der aseptischen Atmosphäre unter Gewinnung eines Wertstoffs, insbesondere eines wirkstoffhaltigen biologischen Materials aus dem geernteten Entwicklungsgut.

15. Verfahren nach Anspruch 14, **dadurch gekennzeichnet, dass** in Schritt b), vorzugsweise nach dem Einsetzen des Lagerbehälters (1) in das Lagergestell (14), eine Sterilisation, vorzugsweise nach einem CIP-Verfahren, erfolgt.
